# FASCICULE DE BREVET EUROPEEN

(11) **EP 1 395 232 B1**
(45) Date de publication et mention de la délivrance du brevet: **18.10.2006**
(21) Numéro de dépôt: 02767522.2
(22) Date de dépôt: 11.06.2002
(51) Int. Cl.: A61K 8/37, A61Q 3/02

(54) **COMPOSITION COSMETIQUE POUR LES ONGLES, SANS PHTALATES, SANS CAMPHRE NI SOLVANT AROMATIQUE**
KOSMETISCHE ZUSAMMENSETZUNG ZUR NAGELPFLEGE, OHNE PHTHALAT, OHNE KAMPFER UND KEINE AROMATISCHE LÖSUNGSMITTEL
COSMETIC COMPOSITION FOR NAILS, FREE OF PHTHALATES, CAMPHOR AND AROMATIC SOLVENT

(30) Priorité: 12.06.2001 FR 0107702
(43) Date de publication de la demande: 10.03.2004
(73) Titulaire: Fiabila, 28130 Maintenon (FR)
(72) Inventeur: MALNOU, Alain, F-27350 Routot (FR); MARTINEZ, Francisco, F-28000 Chartres (FR)
(74) Mandataire: Laget, Jean-Loup
(86) Numéro de dépôt international: PCT/FR2002/001987
(87) Numéro de publication internationale: WO 2002/100322

(56) Documents cités:
- EP-A- 0 455 373
- US-A- 5 145 671

## Description

La présente invention concerne une composition cosmétique pour les ongles, sans phtalates, sans camphre et sans solvants aromatiques et son utilisation en tant que vernis coloré ou de traitement en couche d'adhérence ou en couche incolore de finition.

Typiquement, une formulation de vernis à ongles comporte un polymère filmogène de base, des polymères secondaires, un ou des plastifiant(s) et un ou des solvant(s). Le mélange peut également contenir des pigments, des agents de suspension et des additifs tels que des agents anti-UV, des agents mouillants, des agents hydratants et des parfums.

Toutes ces formulations cherchent à optimiser des performances comme : une application bien arrondie, un temps de séchage court et un très bon brillant. Le formulateur essaie ensuite de garder cet aspect en conférant à la formulation également une bonne résistance à la rayure, à l'usure, à l'écaillage, aux détergents et aux graisses. Ceci doit se traduire par une bonne adhérence et un bon compromis dureté/souplesse dudit vernis.

De nombreuses solutions ont été proposées pour répondre à ces diverses contraintes. Le problème majeur à résoudre est la plastification de la nitrocellulose qui reste la principale résine utilisée dans les vernis à ongles. Les phtalates et le camphre sont de bons plastifiants, mais doivent être remplacés du fait de leur toxicité. Pour des raisons identiques, l'usage de solvants aromatiques doit également être évité.

Les contraintes de réalisation de vernis à ongles sans phtalates, sans camphre, et sans solvants aromatiques ont par conséquent changé les équilibres et les compromis de ces formulations. De nouveaux plastifiants ont été envisagés.

Dans les demandes de brevet EP-A-0 495 373, EP-A-0 613 676 et EP-A-0 613 677, la Société REVLON mentionne divers di et triesters. Dans la demande WO-A-9 820 843, la Société KIRKER utilise un plastifiant spécifique le TXIB (2,2,4-triméthyl 1,3-pentanediol diisobutyrate). La Société L'OREAL plastifie la nitrocellulose avec des polyesters réticulés (EP-A-0 740 930) ou utilise un citrate fluoré (FR-A-2 785 531).

Des mélanges de plastifiants sont aussi proposés, par exemple un mélange de citrate et de toluène sulfonamide (EP-A-0 894 490), un mélange de benzoate de sucrose et de sulfonamide (US-A-4 820 509), ou encore un mélange de sucrose acétate isobutyrate, de butyl benzylbenzoate et de glycéryl tribenzoate (US-A-5 662 891).

Dans ses essais, pour obtenir le meilleur compromis entre dureté, souplesse, séchage, adhérence et durabilité, le demandeur a découvert que des diesters benzoïques de diols peuvent remplacer avantageusement les phtalates.

Ces plastifiants permettent notamment d'avoir, avec les dérivés cellulosiques, des propriétés de brillant, de souplesse, de dureté et d'adhérence très intéressantes sur les ongles.

A cet effet, la composition cosmétique pour les ongles selon l'invention, sans phtalates, sans camphre ni solvants aromatiques, comprend :
a) un polymère filmogène principal cellulosique (A),
b) un diester (B) d'acide benzoïque avec un diol,
   (i) l'acide benzoïque étant soit l'acide benzoïque non substitué, soit un acide benzoïque substitué par un, deux ou trois groupements choisis parmi : -CN, -SCN, -NO₂, -CI, -Br, -F, -OCH₃, -OC₂H₅, -OC₆H₅, -CH=CH₂, un alkyle en C₁-C₆ ramifié ou non, -CH₂CH=CH₂, -NO₂, -NH₂, -OH, -SH et -SO₂NH₂
   (ii) le diol étant choisi parmi :
      ◆ les diols linéaires de formule HO-(CH₂)ₙ-OH (I) dans laquelle n est un entier allant de 4 à 20
      ◆ les diols de formule HO-(CH₂)ₓ-A-(CH₂)_{y}-OH (II)
         x et y étant des entiers de 0 à 4
         A étant un cycle aromatique ou un cycloaliphatique, éventuellement substitué par un alkyle en C₁-C₃₀ linéaire ou ramifié, ou un groupement 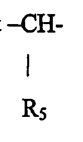 R₅ étant un radical alkyl linéaire ou ramifié en C₁ à C₄.
      ◆ les diols de formule 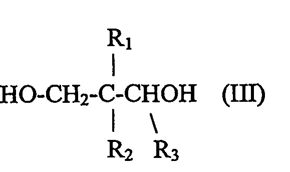 R₁ étant un hydrogène, ou un radical alkyl linéaire ou ramifié en C₁ à C₄
         R₂ étant un hydrogène, ou un radical alkyl linéaire ou ramifié en C₁ à C₄
         R₃ étant un radical alhyl linéaire ou ramifié en C₁ à C₄
      ◆ les diols de formule 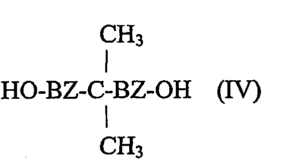 BZ étant un radical benzénique
   c) au moins une résine additive (C).

De manière avantageuse, le polymère filmogène principal A est choisi parmi les nitrocelluloses, les acétobutyrates de cellulose, les acétopropionates de cellulose, les éthers de cellulose et les mélanges de ceux-ci. Le polymère filmogène préféré est la nitrocellulose, en particulier la nitrocellulose à environ 12 %, soluble dans les esters et insoluble dans les alcools. Les grades préférés de nitrocellulose comportent de 120 à 130 motifs cellobiose.

Les acétobutyrates ou acétopropionates de cellulose sont plutôt utilisés dans des vernis incolores de finition où un aspect cristallin est recherché. Les éthers de cellulose, en particulier l'éthyl cellulose, peuvent venir en complément des polymères A ci-dessus pour augmenter la plasticité de la composition.

Le nouveau plastifiant B, diester synthétisé à partir d'un diol et d'un acide benzoïque présente des propriétés équivalentes ou supérieures à celles de phtalates, notamment en ce qui concerne un bon pouvoir de solvatation des polymères cellulosiques, en particulier la nitrocellulose. Les diesters préférés sont les dibenzoates d'hexane diol (en particulier du 1,6-hexanediol), de triméthyl pentane diol (en particulier le 2,2,4-triméthyl 1,3-pentanediol) et de méthyl propane diol (en particulier le di tertio-butyl benzoate de 2-méthyl 1,3-propanediol).

De manière avantageuse, la résine C est choisie parmi les résines (méth)acryliques thermoplastiques, les résines polyesters avec ou sans acides gras mais sans acide phtalique, les condensations de toluène sulfonamide, les résines vinyliques, les résines polyuréthanes et des mélanges de celles-ci.

Les résines, obtenues par condensation de l'éthyl toluène sulfonamide avec le formol ou un composé époxy, sont les résines préférées pour améliorer l'adhérence, en augmentant la polarité du film. Ces résines, riches en solide augmentent l'extrait sec du vernis et lui confèrent un meilleur garnissant et un meilleur arrondi.

D'autres résines peuvent être utilisées pour le même rôle : les résines acryliques et polyesters. Dans la catégorie des acryliques, on trouve des acryliques, des méthacryliques et des styrène/(méth)acryliques. On préférera les résines peu solubles (méthyl et éthyl) de faible poids moléculaire. Certaines résines acryliques modifiées avec des groupes siloxanes ou fluorures peuvent être ajoutées pour leurs qualités de tension et de non rayabilité.

Les résines polyesters sont des condensations de polyacides avec des polyols ou des polyéthylène/polypropylène glycols. Ces résines peuvent être modifiées par des acides gras synthétiques ou des monoglycérides d'huiles végétales. L'anhydride phtalique, l'acide isophtalique et l'acide téréphtalique comme di-acide sont bien entendu écartées pour des compositions cosmétiques sans phtalates.

On utilise des résines courtes en huiles (environ 30 %) avec de l'huile de ricin ou un acide gras synthétique type Cardura E de Shell. Les polyesters cités dans le CTFA comme les copolymères acide adipique / néopentylglycol / anhydride trimellitique ou acide adipique / chdm / ma / néopentylglycol / acide trimellitique sont souvent employés pour les compositions de vernis à ongles.

Les résines vinyliques préférées sont les copolymères à haut poids moléculaire d'acétate de vinyle / chlorure de vinyle partiellement hydrolysé ou le terpolymère chlorure de vinyle / acétate de vinyle / hydroxyléthylacrylate. On peut aussi utiliser un butyral de polyvinyle.

Certaines résines polyuréthanes comme celles décrites dans le CTFA confèrent aux vernis à ongles des propriétés intéressantes, notamment dans le compromis dureté/souplesse.

Un autre plastifiant peut être ajouté au dibenzoate B pour modifier une propriété mécanique souhaitée. A cet effet, la composition selon l'invention peut aussi comprendre d'autres plastifiants et/ou résines plastifiantes D de poids moléculaire inférieur à 1500 environ.

Certaines résines de bas poids moléculaires sont considérées comme des plastifiants externes car elles plastifient les systèmes sans solvater les résines cellulosiques. On peut citer les polyesters adipates, les polyesters sébacates ou les résines d'acrylate de butyle.

De manière avantageuse, la composition selon l'invention peut aussi renfermer au moins un constituant E choisi parmi les colorants, les pigments, les charges, les agents de suspension, les agents anti-UV, les agents anti-oxydants, les actifs comme les vitamines, les protéines, les extraits végétaux, minéraux et/ou animaux, et tout autre ingrédient classique entrant dans un vernis à ongles.

Les pigments, les charges, les nacres artificielles, les paillettes et les cires dures, comme les cires de polypropylène modifiées ou non, sont utilisés pour apporter la couleur et les effets décoratifs aux vernis à ongles.

Les argiles, hectorites ou montmorillonites modifiées, les silices colloïdales, les cires modifiées, les résines d'urée modifiée servent dans les vernis à ongles comme dans les peintures à modifier la rhéologie des systèmes afin de concilier une facilité d'application et le maintien des particules en suspension.

D'autres additifs comme des composés de silicone ou de fluor pour des propriétés de tension et de glissance, les cires dures de granulométrie fine pour le caractère anti-rayure, des composés anti-UV, des agents mouillants pour la stabilité des pigments sont ajoutés pour modifier certaines propriétés bien spécifiques.

Des produits à effets thérapeutiques comme des vitamines, des anti-oxydants, des oligo-éléments, des protéines, des huiles, peuvent être introduits dans certains vernis.

Il a été constaté que les compositions dont la somme des constituants B + D (plastifiants) représente au moins 2 % en poids de la composition totale, de préférence de 6 à 12 % environ, donnaient les meilleurs résultats.

De même le rapport entre le polymère filmogène A et la somme des plastifiants B + D, A/(B+D) est avantageusement compris entre 0,2 et 5, préférentiellement entre 0,6 et 2.

Avantageusement la composition selon l'invention ne contient pas de formaldéhyde. Les résines sont solubilisées dans des solvants non irritants, la composition comprenant au moins un solvant choisi parmi l'acétate d'éthyle, l'acétate de butyle, l'acétate de propyle, l'acétate d'isobutyle, l'éthanol, l'isopropanol et des mélanges de ceux-ci. D'autres solvants comme le diacétonealcool, le méthoxypropanol et certaines cétones peuvent être ajoutés pour ajuster les vitesses d'évaporation et de séchage.

Des agents de rhéologie pouvant être ajoutés à la composition selon l'invention sont de préférence choisis parmi les argiles modifiées du type montmorillonite ou hectorite, les silices pyrogénées hydrophiles ou hydrophobes, les cires modifiées, les résines d'urée modifiée et des mélanges de ceux-ci.

La fabrication de ces vernis est conduite de façon traditionnelle, selon le mode opératoire classique comportant les étapes consécutives suivantes :
- dissolution des résines, plastifiant(s) et divers additifs dans des solvants,
- dispersion des éventuels agents de rhéologie dans ces solutions,
- dispersion des pigments et matières colorantes dans ces solutions,
- préparation d'une base par mélange des solutions et de compléments éventuels d'agents de rhéologie,
- préparation des teintes par ajout à cette base de dispersions pigmentaires, de nacres et de paillettes suivant le standard colorimétrique,
- contrôles de ces produits avant conditionnement.

### EXEMPLES :

La présente invention est illustrée par les exemples de formulations suivantes (tous les pourcentages sont exprimés en poids de composition sèche) :

### EXEMPLE 1 (comparatif) : Formule selon l'Art Antérieur avec phtalate et camphre, sans formol et sans solvants aromatiques

| | |
|---|---|
| Nitrocellulose (à 12,1 %) | 10,6 |
| Résine polyester | 4,6 |
| Phtalate de dibutyle | 4,8 |
| Camphre | 1,4 |
| Solvants | 75,2 |
| Stearalkonium hectorite | 0,9 |
| Pigments | 2,5 |

### EXEMPLE 2 (comparatif) : Formule selon l'Art Antérieur avec phtalate, camphre et formol, sans solvants aromatiques

| | |
|---|---|
| Nitrocellulose | 10,6 |
| Tosylannide/formol | 4,6 |
| Phtalate de dibutyle | 4,8 |
| Camphre | 1,4 |
| Solvants | 75,2 |
| Stearalkonium hectorite | 0,9 |
| Pigments | 2,5 |

### EXEMPLE 3 : Formule sans phtalates ni camphre, sans formol et sans solvants aromatiques

| | |
|---|---|
| Nitrocellulose | 10,5 |
| Dibenzoate d'hexanediol | 8,1 |
| Acryliques | 1,5 |
| Polyester | 4,2 |
| Tosylamide epoxy | 3,0 |
| Solvants | 68,3 |
| Stearalkonium hectorite | 1,0 |
| Pigments | 3,4 |

### EXEMPLE 4 : Formule sans phtalates ni camphre, sans solvants aromatiques et avec formol

| | |
|---|---|
| Nitrocellulose | 10,0 |
| Dibenzoate d'hexanediol | 7,2 |
| Acryliques | 1,5 |
| Tosylamide/formol | 5,2 |
| Polyester | 3,0 |
| Solvants | 68,7 |
| Stearalkonium hectorite | 1,0 |
| Pigments | 3,4 |

### EXEMPLE 5 : Formule sans phtalates ni camphre, sans formol et sans solvants aromatiques

| | |
|---|---|
| Nitrocellulose | 14,5 |
| Dibenzoate de triméthyl pentanediol | 13,1 |
| Solvants | 68,0 |
| Stearalkonium hectorite | 1,0 |
| Pigments | 3,4 |

Les divers vernis, ayant les compositions ci-dessus sont préparés selon le mode opératoire classique décrit en introduction.

Les caractéristiques d'un vernis à ongles étant difficiles à mesurer directement sur les ongles, les tests sont réalisés par comparaison sur d'autres surfaces comme le verre ou l'aluminium (qualité qualicoat 5004 H 24).

Les films sont appliqués à l'aide d'un applicateur de Conway pour une épaisseur humide de 100 micromètres.

Les séchages sont mesurés sur une plaque à 35° C avec la trace que laisse une sphère tournante sur le film de vernis appliqué à 100 micromètres humide sur un carton type Leneta.

Les tests physiques sont effectués après un séchage d'une nuit à 20° C ou de 2 heures à 50° C.

Les épaisseurs sont mesurées sur aluminium selon la norme ISO 2360.

Le brillant est mesuré avec un brillancemètre suivant un angle de 60° selon la norme ISO 2813.

Les duretés sont mesurées sur verre avec un pendule de persoz selon la norme ISO 1522.

La souplesse du film est testée sur aluminium avec un mandrin cylindrique de 3 mm selon la norme ISO 1519.

Des tests de rayabilité, d'usure ainsi que de tenue à l'abrasion humide peuvent être faits avec un appareil de lavabilité des peintures comme décrit dans la norme AFNOR T 30 082.

Les adhérences sont comparées sur verre avec la méthode du quadrillage, sur un film découpé avec un peigne à dents écartées de 1 mm, suivie d'un arrachage au ruban adhésif (cf. norme ISO 2409).

Un test final de durabilité sur l'ongle est organisé sur un échantillon de femmes pour évaluer la tenue du brillant, l'usure et l'écaillage.

Pour les formulations 1 à 5, les principaux résultats obtenus sont les suivants :

| | **N° 1** | **N° 2** | **N° 3** | **N° 4** | **N° 5** |
|---|---|---|---|---|---|
| Séchage | 4,25 min | 3,5 min | 2,5 min | 3,0 min | 3,5 min |
| Dureté | 260 sec | 230 sec | 200 sec | 195 sec | 210 sec |
| Adhérence (0 à 5) | 0/1 | 0 | 0/1 | 0 | 0/1 |
| Souplesse (Cyl. 3 mm) | Passe | Passe | Passe | Passe | Passe |
| Tenue sur ongles | 2,9 jours | 3,4 jours | 3,1 jours | 3,6 jours | 3,5 jours |

On constate que les compositions n° 3 et 5, et n° 4, respectivement sans formol et avec formol, donnent des résultats supérieurs aux compositions correspondantes n° 1 et n° 2, notamment en ce qui concerne la tenue sur les ongles (au moins + 0,2 jour, voire + 0,6 jour pour la composition n° 5) avec un temps de séchage raccourci.

En outre les compositions selon l'invention renfermant le dibenzoate d'hexanediol ou le dibenzoate de triméthyl pentane diol donnent des vernis plus brillants.

## Revendications

1. Composition cosmétique pour ongles, sans phtalate, sans camphre ni solvant aromatique comprenant :
- un polymère filmogène principal cellulosique (A),
- un diester (B) d'acide benzoïque avec un diol,
(i) l'acide benzoïque étant soit l'acide benzoïque non substitué, soit un acide benzoïque substitué par un, deux ou trois groupements choisis parmi : -CN, -SCN, -NO₂, -CI, -Br, -F, -OCH₃, -OC₂H₅, -OC₆H₅, -CH=CH₂, un alkyle en C₁-C₆, ramifié ou non, -CH₂CH=CH₂, -NO₂, -NH₂, -OH, -SH et -SO₂NH₂
(ii) le diol étant choisi parmi :
◆ les diols linéaires de formule HO-(CH₂)ₙ-OH (I) dans laquelle n est un entier allant de 4 à 20
◆ les diols de formule HO-(CH₂)ₓ-A-(CH₂)_{y}-OH (II)
x et y étant des entiers de 0 à 4
A étant un cycle aromatique ou un cycloaliphatique, éventuellement substitué par un alkyle en C₁-C₃₀ linéaire ou ramifié, ou un groupement 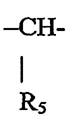 R₅ étant un radical alkyl linéaire ou ramifié en C₁ à C₄.
◆ les diols de formule 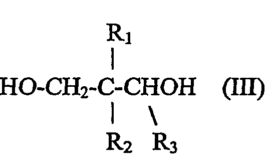 R₁ étant un hydrogène, ou un radical alkyl linéaire ou ramifié en C₁ à C₄
R₂ étant un hydrogène, ou un radical alkyl linéaire ou ramifié en C₁ à C₄
R₃ étant un radical alkyl linéaire ou ramifié en C₁ à C₄
◆ les diols de formule 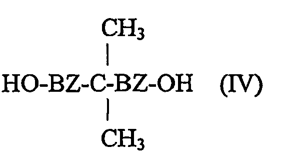 BZ étant un radical benzénique
- au moins une résine additive (C).

2. Composition selon la revendication 1, **caractérisée en ce que** le polymère A est choisi parmi les nitrocelluloses, les acétobutyrates de cellulose, les acétopropionates de cellulose, les éthers de cellulose et les mélanges de ceux-ci.

3. Composition selon l'une quelconque des revendications 1 ou 2, **caractérisée en ce que** la résine C est choisie parmi les résines (méth)acryliques thermoplastiques, les résines polyesters avec ou sans acides gras, les condensations de toluène sulfonamide, les résines vinyliques, les résines polyuréthanes et des mélanges de celles-ci.

4. Composition selon l'une des revendications 1 à 3, **caractérisée en ce qu'**elle comprend d'autres plastifiants et/ou résines plastifiantes D de poids moléculaire inférieur à 1500 environ.

5. Composition selon l'une des revendications 1 à 4, **caractérisée en ce qu'**elle renferme au moins un constituant E choisi parmi les colorants, les pigments, les charges, les agents de suspension, les agents anti-UV, les agents anti-oxydants, les actifs comme les vitamines, les protéines, les extraits végétaux, minéraux et/ou animaux.

6. Composition selon l'une des revendications 4 et 5, **caractérisée en ce que** la somme des constituants B + D représente au moins 2 % en poids de la composition totale, de préférence de 6 à 12 % environ.

7. Composition selon l'une des revendications 1 à 6, **caractérisée en que** le polymère filmogène principal A est la nitrocellulose.

8. Composition selon l'une des revendications 4 à 7, où le rapport entre le polymère filmogène A et la somme des plastifiants B + D, A/(B+D) est compris entre 0,2 et 5, préférentiellement entre 0,6 et 2.

9. Composition selon l'une des revendications 1 à 8, **caractérisée en ce qu'**elle ne contient pas de formaldéhyde.

10. Composition selon l'une des revendications 1 à 9, **caractérisée en ce que** le composant B est le dibenzoate de 2,2,4-triméthyl 1,3-pentane diol.

11. Composition selon l'une des revendications 1 à 9, **caractérisée en ce que** le composant B est un dibenzoate d'hexane diol.

12. Composition selon l'une des revendications 1 à 9, **caractérisée en ce que** le composant B est le di tertio-butyl benzoate de 2-méthyl 1,3-propanediol.

13. Composition selon l'une des revendications 1 à 12, **caractérisée en ce que** la résine C est une résine de condensation entre l'éthyl toluène sulfonamide et le formaldéhyde ou une résine de condensation entre l'éthyl toluène sulfonamide et un composé époxy.

14. Composition selon l'une des revendications 1 à 13, **caractérisée en ce que** la résine C est un polyester sans acide phtalique.

15. Composition selon l'une des revendications 1 à 14, **caractérisée en ce qu'**elle comprend au moins un solvant choisi parmi l'acétate d'éthyle, l'acétate de butyle, l'acétate de propyle, l'acétate d'isobutyle, féthanol, fisopropanol et des mélanges de ceux-ci.

16. Composition selon l'une des revendications 1 à 15, **caractérisée en ce qu'**elle comprend des agents de rhéologie choisis parmi les argiles modifiées du type montmorillonite ou hectorite, les silices pyrogénées hydrophiles ou hydrophobes, les cires modifiées, les résines d'urée modifiée et des mélanges de ceux-ci.

17. Utilisation de la composition selon l'une des revendications 1 à 16 en tant que vernis à ongles, appliqué seul, en une ou plusieurs couches.

18. Utilisation de la composition selon l'une des revendications 1 à 17 en primaire d'adhérence sur l'ongle.

19. Utilisation de la composition selon l'une des revendications 1 à 18 en couche de finition, ou en couche intermédiaire sur l'ongle.

## Claims

1. A cosmetic composition for nails which is devoid of phthalate, of camphor and of aromatic solvent, comprising:
- a main film-forming cellulose polymer (A),
- a diester (B) of benzoic acid with a diol,
(i) the benzoic acid being either non-substituted benzoic acid or a benzoic acid substituted by one, two or three groups selected from among: -CN, -SCN, -NO₂, -Cl, -Br, -F, -OCH₃, -OC₂H₅, -OC₆H₅, -CH=CH₂, a C₁-C₆ alkyl, whether branched or not, -CH₂CH=CH₂, -NO₂, -NH₂, -OH, -SH and -SO₂NH₂,
(ii) the diol being selected from among:
• straight-chain diols of formula HO-(CH₂)ₙ-OH (I) in which n is an integer of from 4 to 20,
• diols of formula HO-(CH₂)ₓ-A-(CH₂)_{y}-OH (II)
x and y being integers from 0 to 4,
A being an aromatic ring or a cycloaliphatic compound, possibly substituted by a straight-chain or branched C₁-C₃₀ alkyl, or a 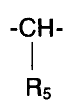 group
R₅ being a straight-chain or branched C₁ to C₄ alkyl radical,
• diols of formula 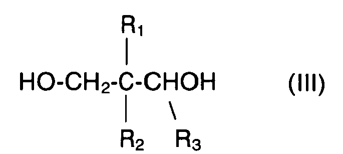 R₁ being a hydrogen, or a straight-chain or branched C₁ to C₄ alkyl radical,
R₂ being a hydrogen, or a straight-chain or branched C₁ to C₄ alkyl radical,
R₃ being a straight-chain or branched C₁ to C₄ alkyl radical,
• diols of formula 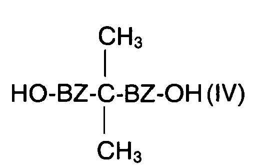 BZ being a benzene radical,
- at least one additive resin (C).

2. A composition according to Claim 1, **characterised in that** the polymer A is selected from among nitrocelluloses, cellulose acetobutyrates, cellulose aceto-propionates, cellulose ethers and mixtures thereof.

3. A composition according to either one of Claims 1 or 2, **characterised in that** the resin C is selected from among thermoplastic (meth)acrylic resins, polyester resins with or without fatty acids, toluene sulphonamide condensation products, vinyl resins, polyurethane resins and mixtures thereof.

4. A composition according to one of Claims 1 to 3, **characterised in that** it comprises other plasticisers and/or plasticising resins D of a molecular weight of less than about 1500.

5. A composition according to one of Claims 1 to 4, **characterised in that** it includes at least one constituent E selected from among colouring agents, pigments, fillers, suspension agents, anti-UV agents, antioxidants, active ingredients such as vitamins, proteins and plant, mineral and/or animal extracts.

6. A composition according to one of Claims 4 and 5, **characterised in that** the total of the constituents B + D represents at least 2% by weight of the total composition, preferably from 6 to 12% approximately.

7. A composition according to one of Claims 1 to 6, **characterised in that** the main film-forming polymer A is nitrocellulose.

8. A composition according to one of Claims 4 to 7, in which the ratio between the film-forming polymer A and the total of the plasticisers B + D, A/(B+D) is between 0.2 and 5, preferably between 0.6 and 2.

9. A composition according to one of Claims 1 to 8, **characterised in that** it does not contain formaldehyde.

10. A composition according to one of Claims 1 to 9, **characterised in that** component B is 2,2,4-trimethyl-1,3-pentanediol dibenzoate.

11. A composition according to one of Claims 1 to 9, **characterised in that** component B is a hexanediol dibenzoate.

12. A composition according to one of Claims 1 to 9, **characterised in that** component B is 2-methyl-1 ,3-propanediol di-tert. butylbenzoate.

13. A composition according to one of Claims 1 to 12, **characterised in that** the resin C is a condensation resin of ethyl toluene sulphonamide and formaldehyde or a condensation resin of ethyl toluene sulphonamide and an epoxy compound.

14. A composition according to one of Claims 1 to 13, **characterised in that** the resin C is a polyester devoid of phthalic acid.

15. A composition according to one of Claims 1 to 14, **characterised in that** it comprises at least one solvent selected from among ethyl acetate, butyl acetate, propyl acetate, isobutyl acetate, ethanol, isopropanol and mixtures thereof.

16. A composition according to one of Claims 1 to 15, **characterised in that** it comprises rheological agents selected from modified clays of montmorillonite or hectorite type, hydrophilic or hydrophobic fumed silicas, modified waxes, resins of modified urea and mixtures thereof.

17. The use of the composition according to one of Claims 1 to 16 as nail varnish, applied alone, in one or more coats.

18. The use of the composition according to one of Claims 1 to 17 as a primer for adhesion to the nail.

19. The use of the composition according to one of Claims 1 to 18 as a top coat, or as an intermediate coat on the nail.

## Patentansprüche

1. Kosmetische Zusammensetzung für Nägel, ohne Phthalat, Campfer oder aromatisches Lösungsmittel, umfassend:
- ein filmbildendes, cellulosehaltiges Hauptpolymer (A),
- einen Diester (B) von Benzoesäure mit einem Diol,
(i) wobei die Benzoesäure unsubstituierte Benzoesäure oder eine Benzoesäure ist, die mit einer, zwei oder drei Gruppen substituiert ist, die ausgewählt ist/sind aus: -CN, -SCN, -NO₂, -Cl, -Br, -F, -OCH₃, -OC₂H₅, -OC₆H₅, -CH=CH₂, verzweigtem oder unverzweigtem C₁-C₆-Alkyl, -CH₂CH=CH₂, -NO₂, -NH₂, -OH, -SH und -SO₂NH₂,
(ii) wobei das Diol ausgewählt ist aus:
• geradkettigen Diolen der Formel HO-(CH₂)ₙ-OH (I),
worin n für eine ganze Zahl von 4 bis 20 steht;
• Diolen der Formel HO-(CH₂)ₓ-A-(CH₂)_{y}-OH (II),
worin x und y ganze Zahlen von 0 bis 4 sind;
A für einen aromatischen oder cycloaliphatischen Ring steht, der gegebenenfalls mit einem geradkettigen oder verzweigten C₁-C₃₀-Alkyl oder mit einer Gruppe 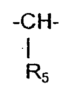 substituiert ist,
wobei R₅ ein geradkettiger oder verzweigter C₁-C₄-Alkylrest ist,
• Diolen der Formel 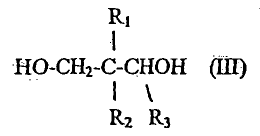
worin
R₁ Wasserstoff oder ein geradkettiger oder verzweigter C₁-C₄-Alkylrest ist,
R₂ Wasserstoff oder ein geradkettiger oder verzweigter C₁-C₄-Alkylrest ist,
R₃ ein geradkettiger oder verzweigter C₁-C₄-Alkylrest ist,
• Diolen der Formel 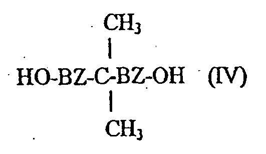 worin BZ ein Benzolrest ist,
- mindestens ein zusätzliches Harz (C).

2. Zusammensetzung nach Anspruch 1, **dadurch gekennzeichnet, daß** das Polymer A aus Nitrocellulosen, Celluloseacetobutyraten, Celluloseacetopropionaten, Celluloseethern und Gemischen davon ausgewählt ist.

3. Zusammensetzung nach einem der Ansprüche 1 oder 2, **dadurch gekennzeichnet, daß** das Harz C aus thermoplastischen (Meth)acrylharzen, Polyesterharzen mit oder ohne Fettsäuren, Toluolsulfonamid-Kondensaten, Vinylharzen, Polyurethanharzen und Gemischen davon ausgewählt ist.

4. Zusammensetzung nach einem der Ansprüche 1 bis 3, **dadurch gekennzeichnet, daß** sie weitere Weichmacher und/oder weichmachende Harze D mit einem Molekulargewicht von weniger als etwa 1500 umfaßt.

5. Zusammensetzung nach einem der Ansprüche 1 bis 4, **dadurch gekennzeichnet, daß** sie mindestens einen Bestandteil E umfaßt, der aus Farbstoffen, Pigmenten, Füllstoffen, Suspendiermittein, anti-UV-Mitteln, Antioxidationsmitteln, aktiven Bestandteilen, wie Vitaminen, Proteinen, pflanzlichen, mineralischen und/oder tierischen Extrakten, ausgewählt ist.

6. Zusammensetzung nach einem der Ansprüche 4 und 5, **dadurch gekennzeichnet, daß** die Summe der Bestandteile B + D mindestens 2 Gew.-%, vorzugsweise etwa 6 bis 12 Gew.-%, der Gesamtzusammensetzung ausmacht.

7. Zusammensetzung nach einem der Ansprüche 1 bis 6, **dadurch gekennzeichnet, daß** es sich bei dem filmbildenden Hauptpolymer A um Nitrocellulose handelt.

8. Zusammensetzung nach einem der Ansprüche 4 bis 7, wobei das Verhältnis zwischen dem filmbildenden Polymer A und der Summe der Weichmacher B + D, A/(B+D) im Bereich von 0,2 bis 5, vorzugsweise von 0,6 bis 2, liegt.

9. Zusammensetzung nach einem der Ansprüche 1 bis 8, **dadurch gekennzeichnet, daß** sie kein Formaldehyd enthält.

10. Zusammensetzung nach einem der Ansprüche 1 bis 9, **dadurch gekennzeichnet, daß** der Bestandteil B das Dibenzoat von 2,2,4-Trimethyl-1,3-pentandiol ist.

11. Zusammensetzung nach einem der Ansprüche 1 bis 9, **dadurch gekennzeichnet, daß** der Bestandteil B ein Dibenzoat von Hexandiol ist.

12. Zusammensetzung nach einem der Ansprüche 1 bis 9, **dadurch gekennzeichnet, daß** der Bestandteil B das Di(t-Butylbenzoat) von 2-Methyl-1,3-propandiol ist.

13. Zusammensetzung nach einem der Ansprüche 1 bis 12, **dadurch gekennzeichnet, daß** das Harz C ein Kondensationsharz aus Ethyltoluolsulfonamid und Formaldehyd oder ein Kondensationsharz aus Ethyltoluolsulfonamid und einer Epoxidverbindung ist.

14. Zusammensetzung nach einem der Ansprüche 1 bis 13, **dadurch gekennzeichnet, daß** das Harz C ein Polyester ohne Phthalsäure ist.

15. Zusammensetzung nach einem der Ansprüche 1 bis 14, **dadurch gekennzeichnet, daß** sie mindestens ein Lösungsmittel umfaßt, das aus Ethylacetat, Butylacetat, Propylacetat, Isobutylacetat, Ethanol, Isopropanol und Gemischen davon ausgewählt ist.

16. Zusammensetzung nach einem der Ansprüche 1 bis 15, **dadurch gekennzeichnet, daß** sie rheologische Mittel umfaßt, die aus modifizierten Tonen vom Typ Montmorillonit oder Hectorit, pyrogenen, hydrophilen oder hydrophoben Kieselerden, modifizierten Wachsen, modifizierten Harnstoffharzen und Gemischen davon ausgewählt sind.

17. Verwendung der Zusammensetzung nach einem der Ansprüche 1 bis 16 als Nagellack, der alleine in einer oder mehreren Schichten aufgetragen wird.

18. Verwendung der Zusammensetzung nach einem der Ansprüche 1 bis 17 als Primärhaftvermittler auf dem Nagel.

19. Verwendung der Zusammensetzung nach einem der Ansprüche 1 bis 18 als Oberschicht oder Zwischenschicht auf dem Nagel.
